(19) **Europäisches Patentamt**

**European Patent Office**

**Office européen des brevets**

(11) **EP 0 316 469 B2**

(12) **NEUE EUROPÄISCHE PATENTSCHRIFT**

(45) Veröffentlichungstag und Bekanntmachung des Hinweises auf die Entscheidung über den Einspruch:
**25.11.1998 Patentblatt 1998/48**

(51) Int. Cl.⁶: **A61B 17/39**

(45) Hinweis auf die Patenterteilung:
**09.08.1995 Patentblatt 1995/32**

(21) Anmeldenummer: **87116954.6**

(22) Anmeldetag: **17.11.1987**

(54) **Hochfrequenz-Chirugiegerät zum Schneiden und/oder Koagulieren biologischer Gewebe**

High frequence surgical device to cut and/or coagulate biological tissues

Appareil chirurgical haute fréquence pour couper et/ou coaguler les tissus biologiques

(84) Benannte Vertragsstaaten:
**DE FR GB IT NL**

(43) Veröffentlichungstag der Anmeldung:
**24.05.1989 Patentblatt 1989/21**

(60) Teilanmeldung:
**95100278.1 / 0 653 192**

(73) Patentinhaber:
**Erbe Elektromedizin GmbH.**
**D-72072 Tübingen (DE)**

(72) Erfinder: **Farin, Günter**
**D-7400 Tübingen (DE)**

(74) Vertreter:
**Endlich, Fritz, Dipl.-Phys.**
**Patentanwalt**
**Meissner, Bolte & Partner**
**Postfach 86 06 24**
**81633 München (DE)**

(56) Entgegenhaltungen:
| | |
|---|---|
| **DE-A- 2 821 498** | **DE-A- 3 531 576** |
| **DE-A- 3 604 823** | **DE-A- 3 623 688** |
| **DE-B- 2 801 833** | **GB-A-  897 961** |
| **US-A- 4 092 986** | **US-A- 4 658 819** |

- **REVUE GENERALE DE L'ELECTRICITE, Nr. 10, Oktober 1981, Seiten 731-737, Paris, FR; R. Brennand "Sécurité des appareils d'électrochirurgie à couranthaute fréquence"**

Bemerkungen:
Teilanmeldung 95100278.1 eingereicht am 17/11/87.

**Beschreibung**

Die Erfindung betrifft ein Hochfrequenz-Chirurgiegerät zum Schneiden und/oder Koagulieren biologischer Gewebe mittels hochfrequenten elektrischen Stromes, mit einem einen Regelverstärker enthaltenden Regelkreis zur automatischen Regelung der Ausgangsspannung des Hochfrequenz-Generators.

Hochfrequenz-Chirurgiegeräte zum Schneiden und/oder Koagulieren biologischer Gewebe mittels hochfrequenten elektrischen Wechselstromes sind seit über 50 Jahren bekannt und gehören seit vielen Jahren zur Ausrüstung chirurgischer Arbeitsplätze verschiedener chirurgischer Fachbereiche.

Die Eigenschaften von sowie die Anforderungen an Hochfrequenz-Chirurgiegeräte sind in nationalen und internationalen Normen, insbesondere in DIN/IEC 601 Teil 2-2, Ausgabe Sept. 1984, definiert und festgelegt.

Wesentlicher Bestandteil aller Hochfrequenz-Chirurgiegeräte sind Hochfrequenzgeneratoren, welche die zum Schneiden und/oder Koagulieren erforderlichen hochfrequenten elektrischen Wechselströme generieren. Um elektrische Reizung von Nerven und Muskeln zu verhindern, soll die Frequenz des Wechselstromes mindestens 300 kHz betragen. Mit Rücksicht auf die Sicherheit des Patienten sowie der Operateure darf die Nenn-Ausgangsleistung von Hochfrequenz-Chirurgiegeräten, gemittelt über eine Sekunde, 400 Watt nicht überschreiten.

Je nach Fachbereich und individuellem Anwendungsfall sind zum Schneiden und/oder Koagulieren mehr oder weniger hohe Leistungen erforderlich. Deswegen sind bekannte Hochfrequenz-Chirurgiegeräte mit Einstellelementen für die Ausgangsleistung ausgestattet. Entsprechend DIN/IEC 601 Teil 2-2 müssen Hochfrequenz-Chirurgiegeräte mit Einstellelementen ausgestattet sein, mit denen die Ausgangsleistung auf nicht mehr als 5 % der Nenn-Ausgangsleistung oder auf 10 Watt abgesenkt werden kann, je nachdem, was kleiner ist.

Bei einem bekannten Hochfrequenz-Chirurgiegerät der eingangs genannten Art mit automatischer Regelung der Intensität der elektrischen Lichtbogen zwischen der aktiven Elektrode und dem zu schneidenden oder zu koagulierenden Gewebe, wird zur Feststellung des Ausmaßes der Lichtbogen ein davon abhängiges elektrisches Signal über ein Filter abgeleitet, welches mindestens eine Frequenz der von den elektrischen Lichtbogen erzeugten nichtharmonischen Frequenzen der Grundfrequenz des Hochfrequenz-Oszillators aus der elektrischen Spannung oder aus dem elektrischen Strom im Ausgang durchläßt. Dabei kann abwechselnd ein Sollwertgeber für die Intensität der Schneideleistung oder ein Sollwertgeber für die Intensität der Koagulationsleistung eingeschaltet werden.Ferner wird die Ausgangsleistung des Leistungsverstärkers durch das Ausgangssignal des Regelverstärkers mittels eines Amplitudenmodulators so gesteuert, daß die Intensität der Lichtbogen auf den Sollwert des betreffenden Sollwertgebers geregelt wird (EP-A- 219 568).

Es ist seit der Einführung der Hochfrequenzchirurgie vor über 50 Jahren bekannt, daß die Leistung, welche Hochfrequenz-Chirurgiegeräte im biologischen Gewebe erzeugen, von verschiedenen Parametern abhängig ist. Die wichtigsten leistungsbestimmenden Parameter sind die Leerlaufspannung und der Innenwiderstand des jeweiligen Hochfrequenzgenerators sowie der elektrische Widerstand des biologischen Gewebes beziehungsweise der Lastwiderstand. Um den Operateuren die Abhängigkeit der vom Hochfrequenz-Chirurgiegerät abgegebenen Leistung vom Lastwiderstand deutlich zu machen, müssen Hersteller von Hochfrequenz-Chirurgiegeräten entsprechend Artikel 6.8.3 DIN/IEC 601 Teil 2-2 in der technischen Beschreibung der Geräte Diagramme angeben, die die abgegebene Leistung bei voller und halber Einstellung des Leistungsstellers über dem Lastwiderstandsbereich von 50 bis 2000 Ohm darstellen, und zwar jeweils für alle Betriebsarten, wie Schneiden, Koagulieren und koagulierender Schnitt. Außerdem müssen auch Diagramme angegeben werden, die die abgegebene Leistung als Funktion der Position des Leistungsstellers für einen anzugebenden Lastwiderstand im Bereich von 50 bis 2000 Ohm für die oben aufgeführten Betriebsarten darstellen.

Dem Operateur helfen die oben genannten Diagramme bestenfalls bei der Auswahl eines für seine spezielle Anwendung geeigneten Hochfrequenz-Chirurgiegerätes. Während der Anwendung, insbesondere wenn dasselbe Hochfrequenz-Chirurgiegeräte für verschiedene Operationen eingesetzt wird, helfen diese Diagramme dem Operateur nicht. Die während des Schneidens und/oder Koagulierens erforderliche Leistung ist nicht, wie oft fälschlich angenommen wird, konstant, sondern von vielen verschiedenen Parametern, wie beispielsweise Tiefe und Geschwindigkeit des Schnittes, Größe der effektiven Kontaktfläche zwischen Koagulationselektrode und Gewebe, Temperatur des koagulierenden Gewebes und physikalische Eigenschaften des zu schneidenden und/oder zu koagulierenden Gewebes, abhängig. In der Praxis ist weder die für den jeweiligen Schneide- und/oder Koagulationsvorgang erforderliche Leistung noch die jeweils vom Hochfrequenz-Chirurgiegerät in jedem Zeitpunkt des Schneide- und/oder Koagulationsvorganges gelieferte Leistung konstant. Dieser Umstand erschwert die Anwendung der Hochfrequenzchirurgie insofern, als die Reproduzierbarkeit der Schneide- und/oder Koagulationseffekte zur Erzielung konstanter Qualität dieser Effekte sehr schwierig ist.

Trotz dieser Problematik sind seit mehreren Jahren mehr und mehr Hochfrequenz-Chirurgiegeräte bekannt geworden, die mit digitalen Ziffernanzeigen ausgestattet sind, welche Leistungen anzeigen, wobei jedoch nicht die jeweils vom Hochfrequenz-Chirurgiegerät abgegebene Leistung, sondern meistens die theoretisch bei Leistungsanpassung mögliche Leistung angezeigt wird. Eine derartige Anzeige nützt dem Operateur jedoch nicht mehr und nicht weniger als eine dimensionslose Skala am Leistungseinsteller, weswegen DIN/IEC 601 Teil 2-2 diesbezüglich auch vorschreibt, daß der Leistungseinsteller für die Ausgangsleistung mit einer Skala oder Anzeigevorrichtung ausgestattet sein muß, die die abgegebene Hochfrequenzleistung in relativen Einheiten anzeigt. Eine Einteilung der Skala für die Ausgangsleistung in 10 Hauptintervallen wird hierin außerdem empfohlen.

Die Problematik der Leistung bei Hochfrequenz-Chirurgiegeräten wird ausführlich beschrieben in FARIN, G.: Möglichkeiten und Probleme der Standardisierung der Hochfrequenzleistung, in: Hochfrequenzdiathermie in der Endoskopie, herausgegeben von G. Lux und K. Semm, Springer-Verlag, Berlin, Heidelberg, 1987.

Da bei konventionellen Hochfrequenz-Chirurgiegeräten eine optimale Übereinstimmung der von Schnitt zu Schnitt beziehungsweise von Koagulation zu Koagulation und auch während jedes einzelnen Schneideund/oder Koagulationsvorganges erforderlichen Leistung und der vom Gerät gelieferten Leistung fast nie erreicht wird, schwankt die Qualität der Schnitte und/oder Koagulationen mehr oder weniger stark.

Aus der deutschen Offenlegungsschrift DE 3531576 A ist ein Elektrochirurgiegenerator bekannt, welcher einige der oben dargestellten Probleme dadurch lösen soll, daß die Ausgangsleistung dieses Generators in einem mehr oder weniger großen Lastwiderstandsbereich automatisch auf eine einstellbare Sollausgangsleistung konstant geregelt wird. Diese Lösung widerspricht der praktischen Erfahrung, daß die während Schneide- und/oder Koagulationsvorgängen erforderliche Leistung nicht konstant, sondern von verschiedenen, bereits oben aufgeführten Parametern abhängig ist.

Aus dieser Problematik resultiert zusätzlich das Problem, daß eine automatische Überwachung, ob das Hochfrequenz-Chirurgiegerät mehr oder weniger Leistung liefert, als für den jeweiligen Zweck erforderlich, nicht möglich ist. Das hat in der Praxis zur Folge, daß mehr Gewebe thermisch geschädigt wird als notwendig, oder daß der gewünschte Effekt ausbleibt. Bei kritischen Operationen, wie beispielsweise im Bereich der operativen Endoskopie, kann dies, wie die Erfahrung zeigt, zu schweren Komplikationen führen.

Aus der amerikanischen Patentschrift US 4,092,986 ist ein Hochfrequenz-Chirurgiegerät bekannt, bei welchem ein Rückkoppelungskreis vom Ausgang zum Eingang des Oszillators vorhanden ist, um die gewählte Ausgangsspannung unabhängig vom Lastwiderstand auf einen konstanten Pegel zu halten. Hierfür wird ein automatischer Regelkreis in an sich bekannter Wirkungsweise vorgestellt, bestehend aus einem Generator, dessen Istwert der Ausgangsspannung mittels eines Detektors ermittelt und mit einem Sollwert verglichen wird, wobei Abweichungen des Istwertes vom Sollwert so auf den Generator einwirken, daß diese Abweichung möglichst klein ist.

Diese Aufgabe wird mit der in US-A-4 092 986 beschriebenen Einrichtung jedoch nur unzureichend gelöst. Der Detektor, welcher den Istwert der Ausgangsspannung ermitteln soll, besteht aus einer Spule 132, welche an den magnetischen Kreis 34 des Resonanzkreises des Oszillators angekoppelt ist. Eine Proportionalität zwischen der Ausgangsspannung des Generators an den Ausgangsbuchsen 122 und dem Ausgangssignal dieses Detektors ist insofern nur unzureichend vorhanden, als der Spannungsabfall an dem bei Hochfrequenz-Chirurgiegeräten allgemein üblichen Kondensator 118 im Anwendungsstromkreis, welcher niederfrequente Ströme und die hierdurch möglichen Reizungen von Nerven und Muskeln verhindern soll und welcher nach der internationalen Norm IEC 601 Teil 2-2 nicht größer als 5000 pF sein darf, nicht berücksichtigt wird. Ein derartiger Kondensator hat beispielsweise bei 500 kHz 63,7 Ohm und bei 300 kHz beispielsweise 106 Ohm Widerstand.

Da bei Schneide- und Koagulationsvorgängen Stromstärken von einigen mA bis zu etwa 2000 mA vorkommen, ergeben sich Schwankungen der Ausgangsspannung bis zu etwa 200 Volt. Bei speziellen Anwendungen, bei denen, wenn auch kurzzeitig, noch größere Stromstärken entstehen, schwankt die Ausgangsspannung entsprechend stärker. Hierdurch wird die gewünschte Reproduzierbarkeit der Schneideund/oder Koagulationsqualität nicht erreicht.

Es ist Aufgabe der Erfindung, Hochfrequenz-Chirurgiegeräte derart zu gestalten, daß die automatische Regelung der HF-Ausgangsspannung im Vergleich zu der aus US-A-4 092 986 bekannten Regeleinrichtung verbessert wird. Ferner sollen die oben aufgeführten Parameter, welche die für Schneide- und/oder Koagulationsvorgänge erforderliche Leistung beeinflussen, weitestgehend automatisch berücksichtigt werden, so daß die Qualität der Schnitte sowie der Koagulationen unabhängig wird von dieses Parametern. Ferner soll ermöglicht werden, die Hochfrequenz-Chirurgiegeräte mit Anzeigeeinrichtungen auszustatten, welche dem Operateur vor und während des Schneidens und/oder Koagulierens zuverlässig relevante, vom Hochfrequenz-Chirurgiegerät bestimmte Parameter anzeigen, die mit der Qualität des Schnittes und/oder der Koagulation korrelieren. Ferner soll ermöglicht werden, die Hochfrequenz-Chirurgiegeräte mit Sicherheitseinrichtungen auszustatten, welche die Einhaltung der für Schneide- und/oder Koagulagionsvorgänge relevanten Parameter überwachen und Abweichungen von Sollwerten dieser Parameter signalisieren.

Diese Aufgabe wird erfindungsgemäß durch den Gegenstand des Patentanspruches 1 gelöst. Vorteilhafte Weiterbildungen der Erfindung sind Gegenstand der abhängigen Ansprüche.

Die Erfindung geht von der Erkenntnis aus, daß zum Schneiden und/oder Koagulieren biologischer Gewebe mittels hochfrequenten elektrischen Wechselstromes zwar Leistung erforderlich, die hierfür erforderliche, von Schnitt zu Schnitt, von Koagulation zu Koagulation und während jedes Schneide- und/oder Koagulationsvorganges mehr oder weniger schwankende Leistung jedoch bei konventionellen Hochfrequenz-Chirurgiegeräten weder optimal voreingestellt noch während einzelner Schneide- und/oder Koagulationsvorgänge optimal dem zu jedem Zeitpunkt bestehenden Leistungsbedarf entsprechend geliefert werden kann.

Die oben aufgeführten Probleme, welche aus der Diskrepanz zwischen für Schneide- und/oder Koagulationsvorgänge erforderlichen und der von konventionellen Hochfrequenz-Chirurgiegeräten gelieferten Leistung resultieren, werden bei dem erfindungsgemäßen Hochfrequenz-Chirurgiegerät vermieden, indem bei diesem Gerät nicht die Leistung, sondern die Ausgangsspannung als Kriterium für Schneide- und/oder Koagulationseffekte eingestellt kontrolliert und angezeigt wird. Voraussetzung hierfür ist jedoch, daß die Ausgangsspannung des Hochfrequenz-Chirurgiegerätes für Schneide- und/oder Koagulationsvorgänge definitiv eingestellt werden kann und daß die eingestellte Ausgangsspannung bei allen relevanten Lastwiderständen automatisch und ausreichend schnell auf den eingestellten Pegel geregelt wird.

Die Erfindung geht außerdem von der Erkenntnis aus, daß zum Schneiden biologischer Gewebe mittels hochfrequenten elektrischen Wechselstromes eine Mindestspannung von etwa 150 $V_{eff}$ erforderlich ist, um die zum Zünden und Aufrechterhalten elektrischer Lichtbogen zwischen Schneideelektrode und Gewebe erforderliche elektrische Feldstärke zu erreichen. Wichtig in diesem Zusammenhang ist auch die Beobachtung, daß bereits geringe Erhöhungen der Ausgangsspannung über die Mindestspannung hinaus deutlich die Schnittqualität ändern, und zwar in der Weise, daß die Intensität der elektrischen Lichtbogen deutlich größer wird und damit auch der Koagulationsgrad der Schnittflächen während des Schneidens. Wichtig in diesem Zusammenhang ist insbesondere auch die Beobachtung, daß die Schnittqualität weitgehend unabhängig von der Schnittgeschwindigkeit und Tiefe des Schnittes wird, wenn die Ausgangsspannung konstant gehalten wird. Bei Koagulationen mit typischen Koagulationselektroden, wie beispielsweise Kugel- oder Plattenelektroden, konnte beobachtet werden, daß die Koagulationsvorgänge und Koagulationsqualität weitgehend unabhängig von der Kontaktfläche zwischen der jeweiligen Koagulationselektrode und dem biologischen Gewebe ist, wenn die Ausgangsspannung konstant gehalten wird. Aber auch hierbei ergeben bereits Änderungen der Ausgangsspannung Infolge der physikalischen Gesetzmäßigkeit, daß die Leistung dem Quadrat der Spannung proportional ist, relativ große Änderungen der Koagulationsqualität.

Die erfindungsgemäße Lösung der oben genannten Aufgabe unterscheidet sich von der oben zitierten bekannten Lösung dadurch, daß die Ausgangsspannung direkt an den Ausgangsbuchsen automatisch kontrolliert und automatisch auf den an einem Sollwertgeber einstellbaren Pegel geregelt wird. Noch besser wäre die Kontrolle und automatische Regelung der elektrischen Spannung direkt zwischen der aktiven Elektrode und der neutralen Elektrode oder gar dem biologischen Gewebe in der Nähe der Schneide- und/oder Koagulationsvorgänge bzw. direkt an den Polen von bipolaren Elektroden.

Die Erfindung berücksichtigt insbesondere das Problem, daß eine definitive Spannungsregelung nur dann möglich ist, wenn die Form der Spannung bzw. deren Anteil harmonischer und/oder nichtharmonischer Frequenzen während aller relevanten Betriebsbedingungen konstant bleibt, da andernfalls das Verhältnis von Spitzenwert zu Effektivwert oder Effektivwert zu einem beliebigen Mittelwert der Spannung nicht konstant wäre. Das erfindungsgemäße Hochfrequenz-Chirurgiegerät kann deswegen weiter verbessert werden, indem es mit einem Hochfrequenzgenerator ausgestattet wird, der eine von allen relevanten Betriebsbedingungen unabhängige konstante Spannungsform, vorzugsweise eine reine Sinusform, erzeugt.

Das erfindungsgemäße Hochfrequenz-Chirurgiegerät bietet nicht nur den Vorteil reproduzierbarer und konstanter Qualität von Schneide- und/oder Koagulationsvorgängen, sondern darüber hinaus auch den Vorteil, daß die Ausgangsspannung vor und während Schneide- und/oder Koagulationsvorgängen automatisch kontrolliert und auf einer elektronischen Anzeigeeinrichtung angezeigt werden. So werden der Istwert der Ausgangsspannung mit dem Sollwert der Ausgangsspannung automatisch verglichen und bei beliebig wählbaren Toleranzgrenzen optische und/oder akustische Signale erzeugt sobald die gewählten Toleranzgrenzen überschritten sind. Ein Über-oder Unterschreiten beliebig wählbarer Toleranzgrenzen der Abweichung des Istwertes vom Sollwert der Ausgangsspann kann auch zur automatischen Abschaltung der Hochfrequenzgenerators verwendet werden. Die Anzeige des Istwertes und/oder des Sollwertes der Ausgangsspannung ermöglicht sowohl eine Voreinstellung der Ausgangsspannung vor dem Einsatz des Gerätes zum Schneiden und/oder Koagulieren als auch eine Kontrolle der Ausgangsspannung während Schneide- und/oder Koagulationsvorgängen.

In weiterer Ausgestaltung der Erfindung können Hochfrequenz-Chirurgiegeräte mit mehreren Sollwertgebern ausgestattet werden, welche verschiedene Sollwerte liefern. Auf diese Weise können verschiedene Schneide- und/oder Koagulationsqualitäten vorprogrammiert und je nach Bedarf über verschiedene Tasten am Elektrodengriff, verschiedene Pedale oder beispielsweise über Tasten auf der Frontplatte des Hochfrequenz-Chirurgiegerätes abgerufen werden. Für diese Ausgestaltung bieten integrierte Halbleiterschaltungen und Mikroprozessoren einfach realisierbare Möglichkeiten.

Anhand schematischer Zeichnungen wird das erfindungsgemäße Hochfrequenz-Chirurgiegerät näher beschrieben. Es zeigen:

**Fig.** 1 Eine schematische Darstellung eines Ausführungsbeispieles eines erfindungsgemäßen Hochfrequenz-Chirurgiegerätes.
**Fig.** 2 Eine schematische Darstellung eines verbesserten erfindungsgemäßen Hochfrequenz-Chirurgiegerätes.
**Fig.** 3 Eine vorteilhafte Ausgestaltung eines erfindungsgemäßen Hochfrequenz-Chirurgiegerätes.
**Fig.** 4 Eine weitere vorteilhafte Ausgestaltung eines erfindungsgemäßen Hochfrequenz-Chirurgiegerätes

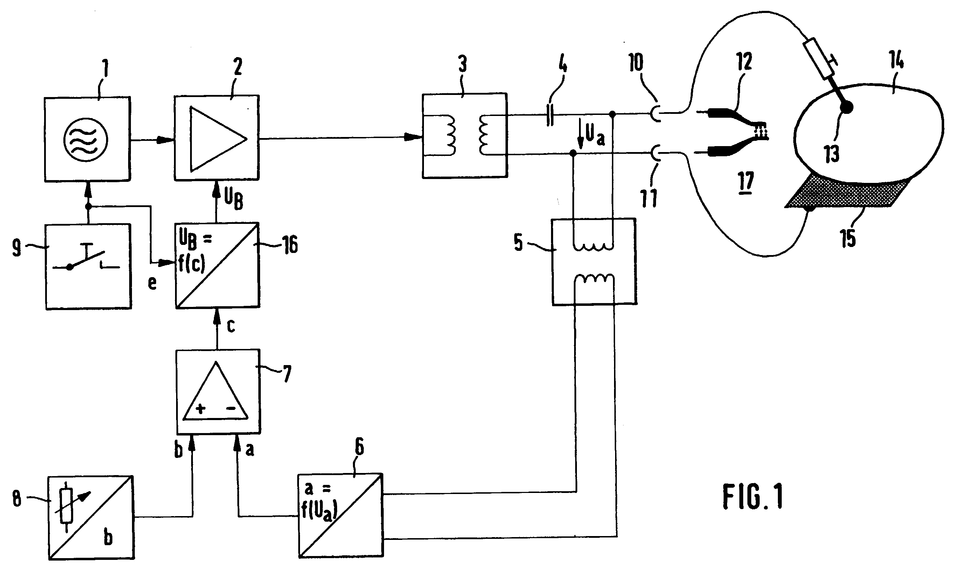

Figur 1 zeigt, als Blockschema dargestellt, die wesentlichsten Bestandteile eines erfindungsgemäßen Hochfrequenz-Chirurgiegerätes. Die an den Ausgangsbuchsen 10,11 anliegende Ausgangsspannung $U_a$ wird über einen Trenntransformator 5 einem Spannungswandler 6 zugeführt, welcher ein der Ausgangsspannung $U_a$ proportionales elektrisches Signal $a = f(U_a)$ liefert. Ist die Ausgangsspannung an den Ausgangsbuchsen 10,11 nicht rein sinusförmig, sondern enthält sie einen nicht vernachlässigbaren Anteil harmonischer und/oder nichtharmonischer Frequenzen der Grundfrequenz und/oder ist die Ausgangsspannung $U_a$ in der Amplitude moduliert und sind diese Abweichungen von der reinen Sinusform außerdem nicht konstant, sondern abhängig von den Betriebsbedingungen, so muß geprüft werden, ob das elektrische Signal a dem Spitzenwert, dem Effektivwert oder einem Mittelwert der Ausgangsspannung $U_a$ proportional sein soll. Dementsprechend muß der Spannungswandler 6 gewählt werden. Geeignete Spannungswandler, welche ein dem Spitzenwert, dem Effektivwert oder ein einem Mittelwert der Ausgangsspannung $U_a$ proportionales Signal $a = f(U_a)$ liefern, sind jedem Fachmann bekannt und werden deswegen hier nicht detaillierter beschrieben.

Der Trenntransformator 5 ist erforderlich um eine ausreichende elektrische Isolationsstrecke zwischen Anwendungsstromkreis 17 sowie interner Betriebsspannungen des Hochfrequenz-Chirurgiegerätes einerseits und Erdpotential andererseits zu gewährleisten.

Diese erforderliche elektrische Isolation kann jedoch auch mit anderen Bauelementen, wie beispielsweise mittels Optokopplern, realisiert werden, wobei beispielsweise eine Glühbirne an die Ausgangsspannung $U_a$ angeschlossen wird und deren Licht beispielsweise einem Fotoelement, einem Fototransistor oder einem Fotowiderstand zugeführt wird, so daß Helligkeitsschwankungen der Glühbirne infolge Schwankungen der Ausgagngsspannung $U_a$ zu proportionalen

elektrischen Signalen am Fotoelement, Fototransistor oder Fotowiderstand führen. Ein derartiger Spannungswandler würde ein dem Effektivwert von $U_a$ proportionales elektrisches Signal liefern.

Das elektrische Signal $a = f(U_a)$ wird in ansich bekannter Weise einem Regelverstärker 7 zugeführt, dem auch ein elektrisches Signal b zugeführt wird, welches den Sollwert der Ausgangsspannung $U_a$ bestimmt. Der Sollwertgeber 8 kann so gestaltet sein, daß er entweder ein über der Zeit konstantes, einstellbares Signal oder ein über der Zeit beliebig in der Amplitude moduliertes Signal b liefert. Je nachdem, wie groß oder klein die Regelzeitkonstante des gesamten Regelkreises ist, folgt die Ausgangsspannung $U_a$ dem Sollwertsignal b mehr oder weniger proportional. Das Ausgangssignal $c = f(b-a)$ des Regelverstärkers 7 wird einem elektronisch gesteuerten Netzteil (16), welches den Vertärker 2 versorgt, zugeführt, wo es die Betriebsspannung $U_B$ des Verstärkers 2 in der Weise steuert, daß die Ausgangsspannung $U_a$ dem Signal b des Sollwertgebers 8 proportional ist.

Die Rückkopplung eines der Ausgangsspannung $U_a$ an den Ausgangsbuchsen 10, 11 des Hochfrequenz-Chirurgiegerätes proportionalen Signales $a = f(U_a)$ zum Zwecke der automatischen Regelung dieser Ausgangsspannung auf den durch das Sollwertsignal b bestimmten Sollwert ist insofern wichtig, als der elektrische Strom im Anwendungsstromkreis 17 sowohl bei Koagulations- als auch insbesondere bei Schneidevorgängen sehr stark schwankt, wodurch z.B. am Kondensator 4 mehr oder weniger große Spannungsabfälle, wie bereits oben dargestellt, entstehen.

Der Vollständigkeit halber sind in Figur 1 auch ein Oszillator 1, welcher mittels eines Tasters oder Pedals 9 aktiviert wird, sowie schematisch biologisches Gewebe 14 sowie eine aktive 13 und eine neutrale Elektrode 15 und alternativ eine bipolare Elektrode 12 dargestellt. Konventionelle Hochfrequenz-Chirurgiegeräte sind in der Regel mit Hochfrequenzgeneratoren ausgestattet, welche mit Rücksicht auf einen möglichst hohen Wirkungsgrad Endverstärker haben,die in Schalterbetrieb betrieben werden, so daß die Form der Ausgangsspannung $U_a$ mehr oder weniger von einer reinen Sinusform abweicht. Wenn diese Abweichung von der reinen Sinusform außerdem auch noch von der Betriebsbedingung des Hochfrequenz-Chirurgiegerätes abhängig ist, wird die Definition der Proportionalität des elektrischen Signales $a = f(U_a)$ insofern problematisch, als das Verhältnis der Spitzenspannung von $U_a$ zum Effektivwert oder einem Mittelwert von $U_a$ nicht konstant ist. Da die Form von $U_a$ bei konventionellen Hochfrequenz-Chirurgiegeräten z.B. vom Lastwiderstand abhängig ist, muß einerseits für Koagulationsvorgänge, bei welchen die Koagulationselektrode während des gesamten Koagulationsvorganges das zu koagulierende biologische Gewebe 14 berührt, und andererseits für Schneidevorgänge, bei welchen die Schneideelektrode während Schneidevorgängen das biologische Gewebe 14 nicht berührt, sondern der Strom durch elektrische Lichtbogen zwischen Schneideelektrode 13 und biologischem Gewebe 14 fließt, definiert werden, wie die Ausgangsspannung $U_a$ bewertet werden soll. Bei Koagulationsvorgängen, bei denen der Strom direkt ohne Lichtbogen von der Koagulationselektrode 13 in das zu koagulierende biologische Gewebe 14 fließt und es deswegen nur auf den Effektivwert der Ausgangsspannung $U_a$ ankommt, sollte das Signal $a = f(U_a)$ proportional dem Effektivwert der Ausgangsspannung $U_a$ sein. Hierfür eignet sich beispielsweise ein fotoelektrischer Spannungswandler, wie er bereits oben beschrieben ist.

Die Abhängigkeit der Proportionalität des elektrischen Signales a von der Form der Ausgangsspannung $U_a$ kann vermieden werden, indem ein Hochfrequenzgenerator verwendet wird, der bei allen relevanten Betriebsbedingungen eine sinusförmige Ausgangsspannung $U_a$ an die Ausgangsbuchsen 10, 11 liefert, also frei ist von harmonischen und/oder nichtharmonischen Frequenzen der Grundfrequenz. Eine vorteilhafte Ausgestaltung des erfindungsgemäßen Hochfrequenz-Chirurgiegerätes stellt daher die Kombination mit einem Hochfrequenzgenerator dar, welcher möglichst nur die Grundfrequenz, also möglichst keine harmonischen und/oder nichtharmonischen Frequenzen der Grundfrequenz erzeugt.

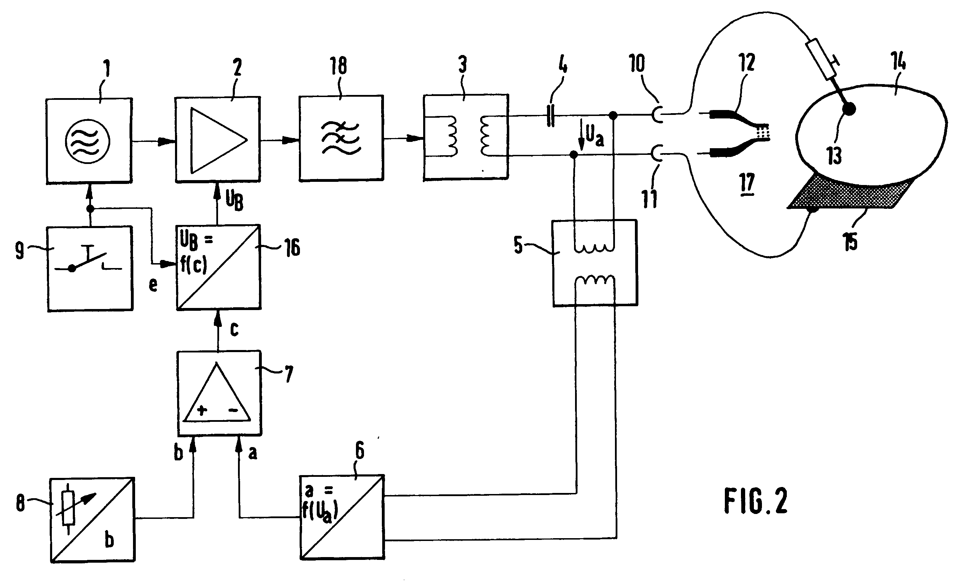

Ein Ausführungsbeispiel eines erfindungsgemäßen Hochfrequenz-Chirurgiegerätes, welches mit einem Hochfrequenzgenerator ausgestattet ist, der eine reine , sinusförmige Ausgangsspannung $U_a$ liefert, ist in Form eines Blockschaltbildes in Figur 2 dargestellt. Zusätzlich zu allen aus Figur 1 bekannten Elementen ist der Ausgang des Hochfrequenzgenerators, bestehend aus einem Oszillator 1, einem Verstärker 2 und einem Ausgangstransformator 3, über ein Tiefpaßfilter 18 geführt, so daß an den Ausgangsbuchsen 10, 11 nur die Grundfrequenz des Oszillators 1 vorhanden ist.

Die automatische Regelung der Ausgangsspannung $U_a$ bietet im Vergleich zu bekannten Hochfrequenz-Chirurgiegeräten, bei denen die Ausgangsspannung entweder nur ungenügend oder gar nicht automatisch geregelt ist, den Vorteil, daß die Ausgangsspannung $U_a$ in einfacher Weise automatisch überwacht und auf einer elektronischen Anzeigeeinrichtung angezeigt werden kann. Da die Höhe der Ausgangsspannung $U_a$ ein wichtiger Parameter für Koagulations- und/oder Schneidevorgänge ist, ist es nicht nur vorteilhaft, diese während Koagulations- und/oder Schneidevorgängen konstant zu regeln, sondern diese bereits vor Beginn der Koagulations- und/oder Schneidevorgänge reproduzierbar und ausreichend genau einstellen zu können.

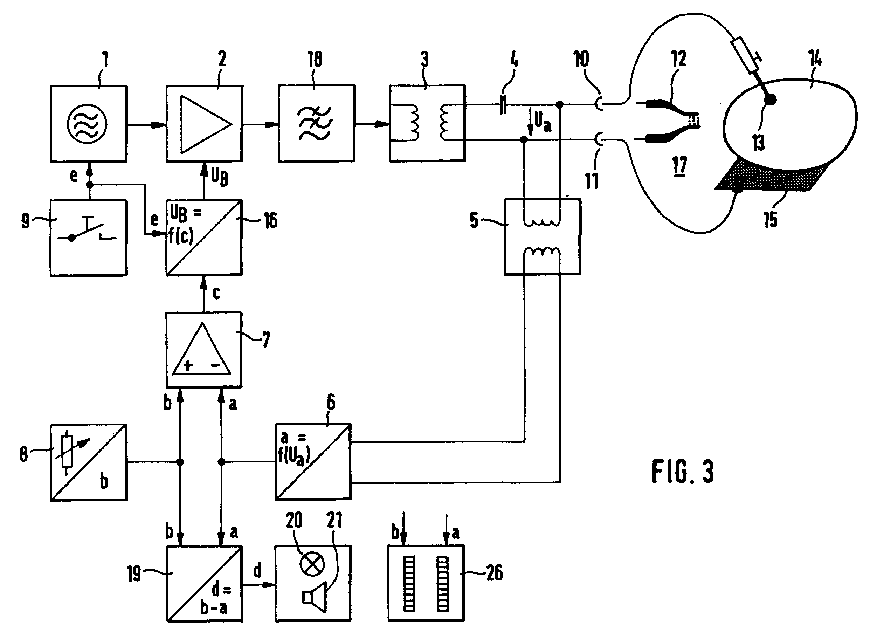

In Figur 3 ist eine diesbezüglich weitere Ausgestaltung des in Figur 2 schematisch dargestellten Hochfrequenz-Chirurgiegerätes ebenfall in Form eines Blockschaltbildes dargestellt. Da das elektrische Signal $a = f(U_a)$ proportional der Ausgangsspannung $U_a$ ist, kann dieses Signal a in einfacher Weise mit dem Signal b, welches den Sollwert von $U_a$ bestimmt, in einem Spannungsvergleicher 19 verglichen werden. Weicht a von b ab, so kann diese Abweichung optisch und/oder akustisch signalisiert werden. Hierbei können beispielsweise positive und/oder negative Abweichungen signalisiert werden, wobei die maximal zulässigen positiven und/oder negativen Abweichungen unabhängig voneinander festgelegt werden können und entweder nur die positiven oder die negativen Abweichungen oder beide optische 20 und/oder akustische 27 Signale auslösen.

Außerdem kann das Signal a und/oder das Signal b auf elektronischen Anzeigeeinrichtungen 26, beispielsweise mittels sogenannter Bar Graphen, angezeigt werden.

Eine weitere vorteilhafte Ausgestaltung des in Figur 3 schematisch dargestellten Hochfrequenz-Chirurgiegerätes bietet die Ausstattung mit mehreren, voneinander unabhängig einstellbaren Sollwertgebern 22,23..., wobei jeder Sollwertgeber über separate Tasten 24,25... eines Elektrodengriffes oder über separate Pedale 24,25... aktivierbar sein kann. Die einzelnen Sollwertgeber können auch über Tasten auf der Frontplatte des Hochfrequenz-Chirurgiegerätes angewählt und über eine Fingertaste am Elektrodengriff oder einem Pedal aktiviert werden.

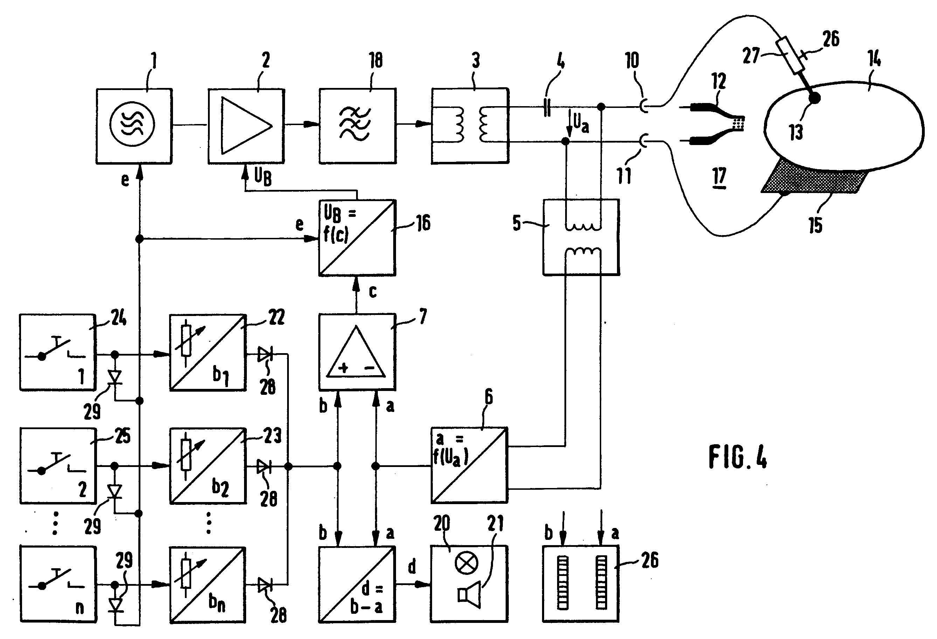

In Figur 4 ist diesbezüglich eine weitere Ausgestaltung des anhand von Fig. 3 beschriebenen Ausführungsbeispieles in Form eines Blockschaltbildes schematisch dargestellt. Dieses in Fig. 4 dargestellte Ausführungsbeispiel ist mit mehreren voneinander unabhängig einstellbaren Sollwertgebern 22, 23 ... n ausgestattet, welche jeweils über separate Tasten am Elektrodengriff und/oder Pedale 24, 25 ... n aktiviert werden. Während bei den vorhergehenden Ausführungsbeispielen entsprechend Fig. 1 bis Fig. 3 der Sollwertgeber ständig ein Sollwertsignal b liefern darf, muß bei mehreren Sollwertgebern entsprechend Fig. 4 darauf geachtet werden, daß der jeweils gewählte Sollwert b1 oder b2 usw. nur solange an den Regelverstärker 7 geliefert wird, wie der jeweilige Sollwertgeber 22, 23 ... n mittels Tasten oder Pedal 24, 25 ... n aktiviert ist. Die verschiedenen Sollwertgeber können jedoch beispielsweise auch per Tasten auf der Frontplatte des Hochfrequenz-Chirurgiegerätes angewählt und mittels nur einer Taste 26 am Elektrodengriff 27 oder per Pedal aktiviert werden. Die Ausgänge der einzelnen Sollwertgeber sind über Dioden 28 gegeneinander entkoppelt. Das Aktivierungssignal e der verschiedenen Tasten 24, 25 ... n wird über die Dioden 29 zusammengefaßt. Das Aktivierungssignal wird nicht nur dem Oszillator 1, sondern auch dem elektronisch gesteuerten Netzteil 16 zugeführt, so daß dieses Netzteil nur dann Betriebsspannung $U_B$ liefert, wenn eine Taste bzw. Pedal 24, 25 ... n betätigt ist.

Bezüglich des Einschwingverhaltens aller Ausführungsbeispiele beim Aktivieren des Hochfrequenzgenerators ist je nach Regelzeitkonstante des Regelkreises darauf zu achten, daß in dem Zeitpunkt, in dem das Sollwertsignal b über den Regelverstärker 7 auf den Verstärker 2 oder auf das elektronisch gesteuerte Netzteil 16 einwirkt, während das Signal a noch nicht am Regelverstärker anliegt, die Ausgangsspannung $U_a$ nicht, wenn auch nur sehr kurzzeitig, unakzeptabel über den gewünschten Sollwert ansteigt. Deswegen ist es beispielsweise zweckmäßig, das elektronisch gesteuerte Netzteil so zu dimensionieren, daß die Anstiegsflanke der Betriebsspannung $U_B$ nicht zu steil ist, sondern mit der Regelzeitkonstante des Regelkreises so abgestimmt ist, daß $U_B$ so ansteigt, daß $U_a$ im Einschaltmoment infolge Regelverzögerung nicht unakzeptabel hoch ansteigen kann.

## Patentansprüche

1. Hochfrequenz-Chirurgiegerät zum Schneiden und/oder Koagulieren biologischer Gewebe mittels hochfrequenten (HF) elektrischen Stromes, mit einem einen Oszillator (1), einen Verstärker (2) und einen Ausgangstransformator (3) enthaltenden HF-Generator, sowie mit einem einen Sollwertgeber (8) zur Erzeugung eines Sollwertsignals (b) und einen Regelverstäker (7) aufweisenden Regelkreis zur automatischen Regelung der Ausgangsspannung des HF-Generators,
dadurch gekennzeichnet,

daß an den Ausgangsbuchsen (10, 11) für den Anwendungsstromkreis (17) oder näher an den Elektroden liegenden Stellen (12, 13, 14, 15) des Anwendungsstromkreises (17) ein Spannungswandler (6) kombiniert mit einer elektrischen Isolation (5) angeschlossen ist, dessen Ausgangssignal als Istwertsignal $(a = f(U_a))$ dem Regelverstärker (7) zugeführt wird, an dem auch ein dem Sollwert der Ausgangsspannung $(U_a)$ entsprechendes Sollwertsignal (b) anliegt,
und daß das Ausgangssignal (c) des Regelverstärkers (7) einem den Verstärker (2) versorgenden elektrisch steuerbaren Netzteil (16) zugeführt wird, um die Betriebsspannung $(U_B)$ für den Verstärker (2) derart zu steuern, daß die Ausgangsspannung $(U_a)$ an den Ausgangsbuchsen (10, 11) oder den näher an den Elektroden liegenden Stellen (12, 13, 14, 15) des Anwendungsstromkreises (17) dem durch das Sollwertsignal (b) bestimmten Sollwert entspricht, wobei weiterhin dem Sollwertsignal (b) und dem Istwertsignal (a) entsprechende Signale einem Spannungsvergleicher (19) zugeführt sind, dem eine Anzeigeeinrichtung (20, 21;) nachgeschaltet ist, um bei Über- oder Unterschreiten vorbestimmter Toleranzgrenzen der Differenz (d) des Sollwertsignals (b) und des Istwertsignals (a) ein optisches Signal und/oder akustisches Signal zur Unterstützung des Operateurs zu erzeugen.

2. Hochfrequenz-Chirurgiegerät nach Anspruch 1,
dadurch gekennzeichnet,

daß die elektrische Isolation in Form eines Trenntransformators (5) oder eines Optokopplers vorgesehen ist.

3. Hochfrequenz-Chirurgiegerät nach einem der vorhergehenden Ansprüche,
dadurch gekennzeichnet,

daß der Hochfrequenz-Generator (1, 2, 3) mit einem Tiefpaßfilter (18) ausgestattet ist, so daß er eine reine sinusförmige Spannung $(U_a)$ an die Ausgangsbuchsen (10, 11) liefert.

4. Hochfrequenz-Chirurgiegerät nach einem der vorhergehenden Ansprüche,
dadurch gekennzeichnet,

daß mehrere voneinander unabhängig einstellbare Sollwertgeber (22, 23 ... n) vorhanden sind, die unabhängig voneinander über Tasten am Elektrodengriff (27) und/oder Pedale (24, 25 ... n)
aktiviert werden können.

5. Hochfrequenz-Chirurgiegerät nach Anspruch 1,
dadurch gekennzeichnet,

daß der Spannungswander (6) derart ausgebildet ist, daß er ein dem Spitzenwert der Ausgangsspannung $(U_a)$ proportionales elektrisches Signal (a) liefert.

6. Hochfrequenz-Chirurgiegerät nach Anspruch 1,
dadurch gekennzeichnet, daß der Spannungswandler (6) derart ausgebildet ist, daß er ein dem Effektivwert der Ausgangsspannung $(U_a)$ proportionales elektrisches Signal (a) liefert.

7. Hochfrequenz-Chirurgiegerät nach Anspruch 1,
dadurch gekennzeichnet,

daß der Spannungswandler (6) derart ausgebildet ist, daß er ein einem Mittelwert der Ausgangsspannung $(U_a)$ proportionales elektrisches Signal (a) liefert.

8. Hochfrequenz-Chirurgiegerät nach Anspruch 4,
dadurch gekennzeichnet,

daß die unabhängig voneinander einstellbaren Sollwertgeber (22, 23 ... n) über Tasten auf der Außenseite des Hochfrequenz-Chirurgiegeräts anwählbar sind.

9. Hochfrequenz-Chirurgiegerät nach einem der vorhergehenden Ansprüche,
dadurch gekennzeichnet,

daß ein Hochfrequenzgenerator (1, 2, 3, 18) vorgesehen ist, der nur die Grundfrequenz ohne harmonische und/oder nicht harmonische Frequenzen der Grundfrequenz erzeugt.

**Claims**

1. A high-frequency surgical instrument for the cutting and/or coagulating of biological tissue by means of high-frequency (HF) electric current, with a HF generator containing an oscillator (1), an amplifier (2) and an output transformer (3), and also with a regulating circuit containing a nominal value setter (8),to produce a nominal value signal (b), and a regulating amplifier (7), for the automatic regulation of the output voltage of the HF generator, characterised in that at the output sockets (10,11) for the application current circuit (17) or points (12,13,14,15) of the application current circuit (17) lying closer to the electrodes, a voltage transducer (6) combined with an electric isolation (5) is connected, the output signal of which is supplied as actual value signal $(a = f(U_a))$ to the regulating amplifier (7), to which also a nominal value signal (b) is supplied corresponding to the nominal value of the output voltage $(U_a)$, and that the output signal (c) of the regulating amplifier (7) is supplied to an electrically controllable power unit (16) supplying the amplifier (2), in order to control the operating voltage $(U_B)$ for the amplifier (2) such that the output voltage $(U_a)$ at the output sockets (10,11) or at the points (12,13,14,15) of the application current circuit (17) lying closer to the electrodes corresponds to the nominal value determined by the nominal value signal (b),
and that signals corresponding to the nominal value signal (b) and the actual value signal (a) are able to be supplied to a voltage comparator (19), after which a display arrangement (20,21) is connected, in order to produce an optical signal and/or an acoustic signal when predetermined tolerance thresholds of the difference (d) of the nominal value signal (b) and of the actual value signal (a) are exceeded or are fallen below, for supporting the operator.

2. A high-frequency surgical instrument according to Claim 1, characterised in that the electric isolation is provided in the form of an isolating transformer (5) or of an opto-coupler.

3. A high-frequency surgical instrument according to one of the preceding claims, characterised in that the high-frequency generator (1,2,3) is equipped with a low-pass filter (18), so that it delivers a pure sinusoidal voltage $(U_a)$ to the output sockets (10,11).

4. A high-frequency surgical instrument according to one of the preceding claims, characterised in that several nominal value setters (22,23 ...n), able to be adjusted independently of each other, are present, which can be activated independently of each other by means of keys on the electrode grip (27) and/or pedals (24,25... n).

5. A high-frequency surgical instrument according to Claim 1, characterised in that the voltage converter (6) is constructed such that it delivers an electrical signal (a) proportional to the peak value of the output voltage $(U_a)$.

6. A high-frequency surgical instrument according to Claim 1, characterised in that the voltage converter (6) is constructed such that it delivers an electrical signal (a) proportional to the effective value of the output voltage $(U_a)$.

7. A high-frequency surgical instrument according to Claim 1, characterised in that the voltage converter (6) is constructed such that it delivers an electrical signal (a) proportional to a mean value of the output voltage $(U_a)$.

8. A high-frequency surgical instrument according to Claim 4, characterised in that the nominal value setters (22,23... n), which are adjustable independently of each other, are able to be selected by means of keys on the exterior of the high-frequency surgical instrument.

9. A high-frequency surgical instrument according to one of the preceding claims, characterised in that a high-frequency generator (1,2,3,18) is provided, which only produces the fundamental frequency without harmonic and/or non-harmonic frequencies of the fundamental frequency.

**Revendications**

1. Appareil chirurgical à haute fréquence pour le découpage et/ou la coalescence de tissus biologiques au moyen de courant électrique à haute fréquence (H.F.), avec un générateur H.F. comprenant un oscillateur (1), un amplificateur (2) et un transformateur de sortie (3), ainsi qu'avec un circuit de régulation pour le réglage automatique de la tension de sortie du générateur H.F. contenant un émetteur de valeur de consigne (8) pour la production d'un signal de valeur de consigne (b) et un amplificateur de régulation (7), caractérisé en ce que, au niveau des bornes de sortie (10,11) pour le circuit du courant d'utilisation (17), ou à des endroits situés plus près des électrodes (12,13,14,15) du circuit du courant d'utilisation (17), on branche un transformateur de tension (6) combiné à une isolation électrique (5), et dont le signal de sortie, en tant que signal de valeur instantanée ($a = f(U_a)$), est acheminé vers l'amplificateur de régulation (7) auquel est également envoyé un signal de valeur de consigne (b) correspondant à la valeur de consigne de la tension de sortie ($U_a$), et en ce que le signal de sortie (c) de l'amplificateur de régulation (7) est envoyé à une partie du réseau (16) à commande électrique qui alimente l'amplificateur (2), afin de commander la tension de service ($U_B$) pour l'amplificateur (2), de telle sorte que la tension de sortie ($U_a$), au niveau des bornes de sortie (10, 11) ou au niveau des endroits situés plus près des électrodes (12,13,14,15) du circuit du courant d'utilisation (17), correspond à la valeur de consigne déterminée par le signal de valeur de consigne (b) et en ce que l'on peut envoyer des signaux correspondant au signal de valeur de consigne (b) et au signal de valeur instantanée (a) à un comparateur de tension (19) auquel est branché un dispositif d'affichage (20,21), afin, lorsque des limites prédéterminées de tolérance pour la différence (d) entre le signal de valeur de consigne (b) et le signal de valeur instantanée (a) ne sont pas atteintes ou sont dépassées, de produire un signal optique et/ou un signal acoustique, pour supporter l'opérateur.

2. Appareil chirurgical à haute fréquence selon la revendication 1, caractérisé en ce que l'isolation électrique est installée sous forme d'un transformateur de séparation (5) ou d'un coupleur optoélectronique.

3. Appareil chirurgical à haute fréquence selon l'une quelconque des revendications précédentes, caractérisé en ce que le générateur à haute fréquence (1,2,3) est équipé d'un filtre passe-bas (18), de sorte qu'il fournit aux bornes de sortie (10,11) une tension (Ua) purement sinusoïdale.

4. Appareil chirurgical à haute fréquence selon l'une quelconque des revendications précédentes, caractérisé en ce qu'il y a plusieurs émetteurs de valeur de consigne (22,23 ..n) réglables indépendamment les uns des autres qui peuvent être activés indépendamment les uns des autres par l'intermédiaire de touches sur le porte-électrodes (27) et/ou de pédales (24,25 ..n).

5. Appareil chirurgical à haute fréquence selon la revendication 1, caractérisé en ce que le transformateur de tension (6) est conçu de telle sorte qu'il fournit un signal électrique (a) proportionnel à la valeur de pointe de la tension de sortie ($U_a$).

6. Appareil chirurgical à haute fréquence selon la revendication 1, caractérisé en ce que le transformateur de tension (6) est conçu de telle sorte qu'il fournit un signal électrique (a) proportionnel à la valeur effective de la tension de sortie ($U_a$).

7. Appareil chirurgical à haute fréquence selon la revendication 1, caractérisé en ce que le transformateur de tension (6) est conçu de telle sorte qu'il fournit un signal électrique (a) proportionnel à une valeur moyenne de la tension de sortie ($U_a$).

8. Appareil chirurgical à haute fréquence selon la revendication 4 caractérisé en ce que les émetteurs de valeur de consigne (22,23, ..n) réglables indépendamment les uns des autres peuvent être choisis par l'intermédiaire de touches situées sur le côté extérieur de l'appareil chirurgical à haute fréquence.

9. Appareil chirurgical à haute fréquence selon l'une quelconque des revendications précédentes, caractérisé en ce qu'il est prévu un générateur à haute fréquence (1,2,3,18) qui ne produit que la fréquence de base sans fréquences harmoniques et/ou non harmoniques de la fréquence de base.

FIG.1

FIG.2

FIG. 3

FIG. 4